# FASCICULE DE BREVET EUROPEEN

(11) **EP 3 160 433 B1**
(45) Date de publication et mention de la délivrance du brevet: **29.07.2020**
(21) Numéro de dépôt: 15742359.1
(22) Date de dépôt: 29.06.2015
(51) Int. Cl.: A61K 8/34, A61Q 13/00, A61K 8/81

(54) **COMPOSITION PARFUMANTE AMÉLIORÉE.**
VERBESSERTE PARFÜMZUBEREITUNG
IMPROVED PERFUME COMPOSITION

(30) Priorité: 30.06.2014 FR 1456116
(43) Date de publication de la demande: 03.05.2017
(73) Titulaire: LvmH Recherche, 45800 Saint-Jean De Braye (FR)
(72) Inventeur: CHEVET, Karine, 45110 Chateauneuf sur Loire (FR); ALARD, Valérie, 45000 Orleans (FR); SOUVIE, Marie-Laure, 45400 Semoy (FR); NOE, Brigitte, 45000 Orleans (FR)
(74) Mandataire: Cabinet Beau de Loménie
(86) Numéro de dépôt international: PCT/FR2015/051770
(87) Numéro de publication internationale: WO 2016/001560

(56) Documents cités:
- FR-A1- 2 162 563
- FR-A1- 2 952 534
- US-A- 3 939 099
- US-A- 6 063 365
- US-A1- 2007 148 213
- US-A1- 2010 267 606

## Description

La présente invention concerne une composition parfumante contenant une forte proportion d'alcool destinée à être appliquée par pulvérisation, directement sur la peau et les cheveux ou bien encore sur les vêtements.

Cette composition peut être une eau de toilette, une eau de Cologne, un extrait de parfum, un esprit de parfum ou une eau de parfum. Elle n'est pas rincée après application.

Plus précisément, l'invention se rapporte à une composition parfumante ayant d'excellentes propriétés telles que la transparence, l'aptitude à la pulvérisation, la fidélité olfactive du concentré de parfum qu'elle contient, et la rémanence de la note parfumée au cours du temps après application.

### ART ANTERIEUR

Un parfum contient généralement un concentré de parfum, de l'éthanol, éventuellement de l'eau, et des additifs tels que des agents solubilisants, des colorants, des agents anti-oxydants et des agents tensio-actifs.

Le concentré de parfum est un constituant essentiel du parfum. Il renferme essentiellement des substances volatiles odorantes qui sont associées, pour conférer au parfum sa note olfactive originale.

La mise au point d'une solution parfumante implique la sélection de la nature et de la quantité de ces différents ingrédients dans le but d'obtenir un jus conforme à la réglementation, ayant des caractéristiques organoleptiques optimales et une bonne stabilité. Les documents US 3,939,099 et FR 2 952 534 décrivent une composition parfumante comprenant au moins une substance parfumante et au moins un polymère vinylpyrrolidone cationique.

Le parfum doit en particulier présenter une stabilité à la lumière et à la chaleur suffisante pour garantir le maintien de la note olfactive dans le conditionnement pendant son stockage jusqu'à la vente du produit, puis pendant toute la durée de son utilisation par le consommateur. Cette stabilité implique principalement la conservation dans le temps de la note olfactive originale et l'absence d'apparition de troubles ou de dépôts dans le flacon.

L'odeur d'un parfum, également appelée « note olfactive » dans la présente demande, est le résultat perçu par l'odorat de de l'émanation des substances volatiles qu'il contient. En jouant sur les degrés de volatilité des substances odorantes, et les seuils de perceptions par l'odorat, on compose un parfum dont la note olfactive évolue au cours des heures suivant l'application sur la peau ou les cheveux.

Ainsi, chaque parfum présente ce que l'on appelle i) une note de tête qui est l'odeur diffusant en premier lors de l'application du parfum ou lors de l'ouverture du récipient le contenant, ii) une note de cœur ou corps qui correspond au parfum complet (émission pendant quelques heures après extinction progressive de la note de tête) et iii) une note de fond qui est l'odeur la plus persistante (émission pendant plusieurs heures après la note de cœur). La persistance de la note de fond correspond à ce que les parfumeurs appellent la rémanence du parfum.

Pour certains concentrés de parfum, on cherchera à augmenter la rémanence de la note olfactive de la solution parfumante, pour prolonger la perception odorante chez le consommateur au cours du temps, une fois la composition parfumante appliquée. On pourra également chercher à conserver l'intensité des notes plus volatiles, telles que les notes de tête et les notes de cœur.

Dans tous les cas, il est essentiel d'incorporer dans les solutions parfumantes, des composés qui ne modifient pas l'ensemble de la senteur, notamment les notes de tête, de cœur et de fond de manière qualitative (odeur perçue) et quantitative (intensité perçue), afin de garantir la fidélité olfactive du concentré de parfum.

Ainsi, de multiples paramètres doivent être ajustés pour obtenir un produit satisfaisant. Or un additif donné peut apporter simultanément à sa fonction principale des propriétés indésirables, si bien que le parfumeur peut être amené à faire des compromis.

Par exemple, l'intensité et la rémanence d'une note olfactive peuvent dépendre de la nature du concentré de parfum, du pourcentage du concentré dans la solution, du taux d'alcool et du temps de macération. La teinte dépend des paramètres de filtration du jus ainsi que de l'ajout de colorants. Un système de protection et de stabilisation du concentré de parfum comprenant des agents antioxydants et des filtres solaires protégeant la solution parfumante de l'action des UV peut être nécessaire pour limiter la détérioration du jus à la lumière et à la chaleur. De plus, l'innocuité toxicologique de la formule et sa bonne compatibilité avec le contenant doivent être vérifiées.

Les principaux retours négatifs des consommateurs au sujet des produits parfumants tels que les eaux de toilette sont souvent leur manque de tenue sur peau : la perception de l'odeur du produit une fois appliqué est jugée trop courte, si bien que le consommateur peut être obligé de réappliquer le produit une ou plusieurs fois au cours de la journée, afin de maintenir la perception de son odeur dans le temps. Aussi, lors de la préparation d'une composition de parfum, on peut être amené à y incorporer des composés qui retardent leur évaporation, tels que des fixateurs ou des agents modificateurs de leur courbe d'évaporation.

Certains fixateurs et certains modificateurs du profil d'évaporation présentent cependant l'inconvénient de modifier les propriétés organoleptiques d'une solution alcoolique du concentré de parfum. Ils peuvent ainsi moduler les notes parfumées du produit parfumant perçues après application du produit sur la peau ou les vêtements. Ils peuvent également laisser au toucher une sensation de gras ou de collant sur la peau, ou modifier l'aspect de la solution en diminuant sensiblement sa transparence ou en augmentant sa viscosité. Ils peuvent enfin diminuer la stabilité à la chaleur et à la lumière de la solution.

Il subsiste donc le besoin de proposer des composés qui augmentent la durée de perception de la note du parfum, sans modifier la couleur et l'identité olfactive du concentré de parfum dont on souhaite préserver les notes. Ces composés doivent également permettre de conserver la transparence de la solution parfumante, pouvoir être pulvérisés à travers un dispositif adapté tel que la buse d'un flacon, et ne pas laisser sur la peau des résidus collants.

Les inventeurs ont trouvé un composé de type polymère répondant à ce cahier des charges complexe. Ceci est d'autant plus surprenant qu'une multitude d'autres polymères ne permettent pas de le remplir. C'est le cas notamment des dérivés méthacrylates qui ont une odeur rédhibitoire, des dérivés de cellulose pour lesquels un compromis entre l'effet prolongateur et le caractère pulvérisable n'a pu être trouvé, les polyvinylpyrrolidones qui conduisent à des solutions trop visqueuses pour être pulvérisables, ou à des solutions pulvérisables mais dont l'effet prolongateur n'est pas significatif. Enfin, d'autres polymères conduisent à des solutions parfumantes qui laissent une sensation de collant sur la peau.

Les inventeurs ont trouvé de façon surprenante qu'un copolymère de vinylpyrrolidone et d'acétate de vinyle permet de conserver et prolonger la note olfactive originale de parfums tout en conservant leur caractère pulvérisable. Ce composé permet ainsi de prolonger la perception odorante des matières présentes dans le concentré de parfum, qui a été dilué dans un alcool.

Les copolymères de vinylpyrrolidone et d'acétate de vinyle ont déjà été utilisés dans le domaine de la cosmétique. Leurs propriétés filmogènes ont été mises à profit dans des produits pour cheveux, tels que des shampooings et des gels, pour faciliter leur maintien et leur mise en forme.

La présente invention propose d'utiliser un copolymère de vinylpyrrolidone et d'acétate de vinyle comme prolongateur de parfum.

### DESCRIPTION DE L'INVENTION

La présente invention a ainsi pour objet une composition parfumante, ladite composition comprenant :
- de 40 à 90% en poids d'éthanol,
- de 0 à 30% en poids d'eau,
- de 3 à 40% en poids d'un concentré de parfum, et
- de 1 à 10% en poids d'un copolymère de vinylpyrrolidone et d'acétate de vinyle, de préférence solide à 25°C, le ratio massique entre le vinylpyrrolidone et l'acétate de vinyle dans le copolymère étant égal à 60:40,
les pourcentages étant exprimés par rapport au poids de la composition.

La composition parfumante peut être constituée :
- de 40 à 90% en poids d'éthanol,
- de 0 à 30% en poids d'eau,
- de 3 à 40% en poids d'un concentré de parfum, et
- de 1 à 10% en poids d'un copolymère de vinylpyrrolidone et d'acétate de vinyle, de préférence solide à 25°C,
les pourcentages étant exprimés par rapport au poids de la composition.

On entend par « composition parfumante » ou « parfum », un produit sous forme liquide destiné à parfumer un individu après sa pulvérisation ou son application sur : la peau, les cheveux ou les vêtements de l'individu. Un tel produit n'est pas rincé après application. Un parfum est généralement appliqué sur la peau.

Par « comprenant », on entend que la composition peut contenir d'autres ingrédients. On préfère que la somme des pourcentages de l'éthanol, de l'eau, du concentré de parfum et du copolymère soit supérieure ou égale à 95% en poids, de préférence encore supérieure à 98% en poids du poids de la composition.

Par « constituée », on entend que la somme des pourcentages de l'éthanol, de l'eau, du concentré de parfum et du copolymère est égale à 100%.

Le copolymère permet d'augmenter la rémanence de la note olfactive de la composition sans changer la note olfactive originale perçue au moment de l'utilisation. Incorporé dans une solution parfumante, ce copolymère ne modifie pas de manière significative la capacité de la solution à être pulvérisée de façon satisfaisante.

La composition est de préférence une formule de parfum pulvérisable, transparente, non collante, stable dans le temps, et stable à la chaleur. La note olfactive de la composition parfumante dans son conditionnement ne varie pas significativement après exposition de celle-ci à la chaleur pendant plusieurs semaines.

Par « pulvérisable », on entend l'aptitude d'une composition à être pulvérisée de façon satisfaisante pour le consommateur, celui-ci s'attendant à ce qu'elle puisse être pulvérisée de façon régulière et répétée en produisant un résultat satisfaisant en terme d'homogénéité de la taille des gouttelettes formées à la pulvérisation, à la surface formée par le nuage de composition pulvérisée à une distance donnée ou encore le résultat obtenu après application sur la peau.

Le copolymère ne modifie pas significativement la note olfactive d'une solution alcoolique d'un concentré de parfum. Il permet également de conserver la note olfactive originale en condition standard de vieillissement accéléré, notamment par exposition à la chaleur.

Aussi, le copolymère améliore les propriétés des compositions parfumantes, en particulier leur rémanence sur la peau ou les cheveux, tout en obtenant une composition transparente que l'on peut pulvériser, et dont les propriétés sensorielles telles que le toucher sont satisfaisantes.

Le copolymère prolonge avantageusement la perception du parfum dans le temps, en particulier les notes de tête et les notes de cœur du parfum, pour un utilisateur qui a pulvérisé la composition sur sa peau ou ses vêtements, cette augmentation étant évaluée par rapport à la même composition ne contenant pas ledit copolymère.

Dans le copolymère, le ratio massique entre le vinylpyrrolidone et l'acétate de vinyle dans le copolymère peut aller avantageusement de 50 :50 à 70 :30. Il est de 60 :40.

La masse moléculaire en poids du copolymère, mesurée selon la méthode de diffraction laser aux petits angles, est de préférence comprise entre 15.000 et 600.000, de préférence encore entre 30.000 et 100.000.. La masse moléculaire en poids du copolymère est par exemple de l'ordre de 40.000 à 50.000.

Le copolymère est de préférence solide à 25°C, et peut se présenter sous la forme d'une poudre. Le nom INCI du copolymère peut être VP/VA Copolymer. Il a de préférence une température de transition vitreuse comprise entre 70 et 115°C, de préférence encore entre 100 et 110°C.

La composition est liquide à 25°C, et se présente avantageusement sous la forme d'une solution des ingrédients qui la composent. Elle peut être hydro-alcoolique ou alcoolique. On entend par « composition hydro-alcoolique », une composition contenant de l'éthanol et de l'eau, et par « composition alcoolique », une composition contenant de l'éthanol et ne contenant pas d'eau. L'éthanol utilisé pour la formulation des compositions est généralement de l'éthanol à 96% en volume (i.e. contenant 4% d'eau). Au sens de la présente invention, lorsque l'on exprime le pourcentage d'eau dans la composition, on entend le pourcentage d'eau que la composition contient i) du fait de l'utilisation d'éthanol à 96% en volume, ou ii) du fait de l'ajout d'eau à de l'éthanol absolu.

Dans le cadre de l'invention, l'expression « de...à » entend inclure les bornes de la plage de valeurs, à la différence de l'expression « entre...et » qui entend exclure les bornes de la plage de valeurs.

La composition est avantageusement sous la forme d'une solution dans laquelle les ingrédients sont miscibles. Dans ce cas, la composition ne se trouve pas sous la forme d'une émulsion eau-dans-huile ou huile-dans-eau.

La composition contient avantageusement de 0 à 0,01% en poids de tensio-actif. Elle en est de préférence exempte. En effet, le copolymère est soluble à la fois dans l'éthanol, le concentré de parfum, et le mélange des deux, si bien qu'il n'est pas nécessaire d'ajouter un agent tensio-actif pour que la composition soit homogène et transparente.

La composition peut être en particulier exempte de tensioactifs polyoxyalkylénés comprenant au moins cinq motifs choisis parmi - CH₂CH(OH)CH₂- et -OCH₂CH₂-, tels que les composés polyoxyéthylénés et les composés polyoxypropylénés. On peut citer parmi ces tensio-actifs les éthers polyoxyalkylénés, comme le POE(10) cetyl éther, les esters polyoxyalkylénés, comme le PEG-40 hydrogenated castor oil ou le POE(20) sorbitan monolaurate, les condensats d'alkylphénols polyoxyéthylénés, les produits de condensation d'oxyde d'éthylène avec le produit de réaction d'oxyde de propylène et de l'éthylène diamine, les alcools polyéthoxylés, les polysorbates, et les diméthicone copolyols.

Le copolymère est avantageusement en une quantité suffisante pour augmenter la rémanence du concentré de parfum dans le temps, une fois la composition appliquée sur la peau ou les cheveux, sans diminuer la transparence et l'aptitude à la pulvérisation de la composition.

Dans un mode de réalisation, le copolymère représente de 1 à 10% en poids de la composition. Au-delà de 10% en poids, le copolymère a un effet négatif : il procure une sensation de collant sur la peau et il empêche la bonne pulvérisation de la composition. Dans un mode de réalisation, le copolymère représente de 2 à 8%, de préférence de 2 à 5% en poids du poids de la composition.

La concentration minimale en copolymère a une valeur choisie dans le groupe consistant en 1%, 2% et 3% en poids du poids de la composition. La concentration maximale en copolymère a une valeur choisie dans le groupe consistant en 4%, 5%, 6%, 7%, 8%, 9% et 10% en poids du poids de la composition.

La composition contient un concentré de parfum. Le concentré de parfum peut être par exemple choisi parmi des composés dont le nom INCI figurant sur la liste des ingrédients de la composition parfumante proposée à la vente est « Parfum ». Un concentré de parfum est un composé ou un mélange de composés au moins partiellement volatil à température ambiante, dont on détecte l'odeur. Le concentré de parfum contient une note choisi dans le groupe constitué des notes de têtes, des notes de cœur, des notes de fond, et leurs mélanges. Le concentré de parfum comprend de préférence majoritairement des notes de têtes et des notes de cœur, et correspond à des parfums de faible rémanence ou de rémanence moyenne.

On entend par faible rémanence ou rémanence moyenne, un parfum dont la perception odorante par un panel expérimenté diminue de plus de moitié au bout de 8 heures après application sur la peau.

Le concentré de parfum est préparé à partir de matières parfumantes naturelles ou synthétiques.

Comme matières parfumantes d'origine naturelle, on peut citer par exemple les extraits de fleurs (lavande, rose, jasmin, ilang-ilang), de tiges et de feuilles (patchouli, géranium, petit-grain), de fruits (coriandre, anis, cumin, genièvre), d'écorces de fruits (bergamote, citron, orange), de racines (angélique, céleri, cardamome, iris, acore), de bois (santal, gaïac, cèdre rose), d'herbes et de graminées (estragon, lemon grass, sauge, thym), de résines et de baumes (galbanum, élémi, benjoin, myrrhe, oliban, opopanax).

Comme matières parfumantes d'origine synthétique, on peut citer par exemple l'acétate de benzyle, le benzoate de benzyle, l'isobutyrate de phénoxyéthyle, l'acétate de p-tert-butylcyclohexyle, l'acétate de citronellyle, le formiate de citronellyle, l'acétate de géranyle, l'acétate de linalyle, l'acétate de diméthyl-benzylcarbinyle, l'acétate de phényléthyle, le benzoate de linalyle, le formiate de benzyle, le glycinate d'éthylméthylphényle, le propionate d'alkylcyclohexyle, le propionate de styralyle et le salicylate de benzyle, le benzyléthyléther, les alcanals linéaires comportant de 8 à 18 atomes de carbone, le citral, le citronellal, le citronellyloxyacétaldéhyde, le cyclamènaldéhyde, l'hydroxycitronellal, le lilial et le bourgeonal, les ionones comme l'alpha-isométhylionone, et la méthylcédrylcétone, l'anéthol, le citronellol, l'eugénol, l'isoeugénol, le géraniol, le linalol, le phényléthylalcool, le terpinéol, les terpènes. Ces composés se présentent souvent sous forme de mélange de deux ou plus de ces substances odorantes.

Par ailleurs, on peut aussi utiliser des huiles essentielles, comme par exemple les essences de sauge, de camomille, de girofle, de mélisse, de menthe, de feuilles de cannelier, de genièvre, de vétiver, d'oliban, de galbanum, de labdanum et de lavandin.

On utilise de préférence comme parfum, seule ou en mélange, l'essence de bergamote, le dihydromyrcénol, le lilial, le lyral, le citronellol, l'alcool phényléthylique, l'alpha-hexylcinnamaldéhyde, le géraniol, la benzylacétone, le cyclamènaldéhyde, le 25 linalol, l'ambroxane, l'indol, l'hédione, la sandelice, les essences de citron, de mandarine et d'orange, le glycolate d'allylamine, le cyclovertal, l'essence de lavandin, l'essence de sauge, le bétadamascone, l'essence de géranium, le salicylate de cyclohexyle, l'acide phénylacétique, l'acétate de géranyle, l'acétate de benzyle, l'oxyde de rose.

Parmi les notes olfactives connues, on peut citer par exemple les parfums hespéridés, les aromatiques, les parfums floraux, les musqués, les parfums fruités, les épicés, les parfums orientaux, les parfums marins, les notes aquatiques, les parfums chyprés, les parfums boisés, les fougères et leurs mélanges

La teneur en éthanol dans la composition peut varier de 40 à 90% en poids, de préférence de 50 à 85% en poids par rapport au poids de la composition.

Dans un premier mode de réalisation, la composition contient de 15 à 25% en poids de concentré de parfum, de 55 à 65% en poids d'éthanol et de 15 à 25% en poids d'eau par rapport au poids de la composition.

Dans un deuxième mode de réalisation, la composition contient de 25 à 35% de concentré de parfum, de 70 à 80% en poids d'éthanol et 0% en poids d'eau ajoutée par rapport au poids de la composition.

Dans un troisième mode de réalisation, la composition contient de 15 à 25% en poids de concentré de parfum, de 75 à 85% en poids d'éthanol et de 0 à 5% en poids d'eau par rapport au poids de la composition.

D'une manière générale, lorsque la composition contient de l'eau, le ratio massique entre l'éthanol et l'eau, est compris entre 65/35 et 98/2, de préférence entre 70/30 et 85/15, et de préférence encore entre 75/25 et 80/20 dans la composition de l'invention.

Le concentré de parfum peut représenter de 6 à 30% en poids de la composition, par exemple de 6 à 15%, ou de 10 à 25%, ou encore de 20 à 30% en poids du poids de la composition. Le concentré de parfum peut représenter de 3% à 40%, par exemple de 10% à 35% ou de 15% à 30% en poids du poids de la composition.

La composition peut contenir outre les ingrédients décrits précédemment au moins un ingrédient cosmétiquement acceptable choisi parmi les colorants, les filtres UV, les actifs cosmétiques, les anti-oxydants et les agents rafraîchissants.

Les colorants sont par exemple: le caramel, Yellow 5, Acid Blue 9/ Blue 1, Green 5, Green 3 / Fast Green FCF 3, Orange 4, Red 4 / Food Red 1, Yellow 6, Acid Red 33 / Food Red 12, Red 40, le carmine de cochenille (Cl 15850, Cl 75470), Ext. Violet 2, Red 6-7, Ferric Ferrocyanide, Ultramarines, Acide Yellow 3 / Yellow 10, Acid Blue 3, Yellow 10. Les colorants liposolubles sont par exemple le rouge Soudan, le D&C Red 17, le D&C Green 6, le bêta-carotène, l'huile de soja, le brun Soudan, le D&C Yellow 11, le D&C Violet 2, le D&C Orange 5, le jaune quinoléine, le rocou.

Les colorants représentent généralement de 0,01 à 1%, de préférence de 0,05 à 0,5%, en poids du poids de la composition parfumante.

Parmi les antioxydants, on peut citer par exemple l'acide ascorbique, le di-tert-butyl-p-hydroxytoluène (encore appelé BHT ou 2,6-di-tert-butyl-p-crésol), le BHA (tert-butyl-4-hydroxyanisole), les tocophérols comme la vitamine E, les dérivés du tocophérol tels que l'acétate de tocophéryle, l'acide gallique et ses dérivés.

La composition peut comprendre en outre un ou plusieurs agents modificateurs de la courbe d'évaporation des molécules présentes dans le concentré de parfum. De tels modificateurs sont choisis parmi les glycols, en particulier parmi les éthers éventuellement hydroxylés ou polyalkylénés, les glycéryl éthers en C4 à C12, les esters, et les esters polyalkylénés.

Un de ces éthers peut correspondre à un composé de formule R-O-(CH(CH₃)-CH₂O)ₐ-(CH₂-CH₂O)_{b}-H dans laquelle a et b sont des nombres entiers tels que la somme de a et b va de 1 à 4, et R est une chaîne aliphatique comprenant de 8 à 18 atomes de carbone.

Un éther peut être par exemple choisi parmi le polyoxyéthylène Glycéryl Monococoate (Cetiol® HE), le dicaprylyl éther (Cétiol® OE), le PPG-11 stéaryl éther (Arlamol® E), le PPG-3 myristyl éther (Tegosoft® APM), et leurs mélanges.

Des glycéryl éthers peuvent être conformes à ceux décrits dans le document US 2003/0216283, notamment le 2-éthylhexyloxy-propanediol.

Un ester peut être le n-hexadécyl-n-nonanoate, le n-octadécyl-n-nonanoate ou le néopentylglycol diisononanoate.

L'invention a également pour objet un flacon muni d'un moyen de pulvérisation et d'un moyen de conditionnement, et contenant la composition décrite précédemment.

Le moyen de pulvérisation peut être une pompe manuelle. Le flacon est de préférence transparent afin de laisser percevoir la composition de l'invention, qui est de préférence elle-même transparente.

Les compositions peuvent être appliquées sous forme de fines gouttelettes au moyen de dispositifs de pressurisation. Ces dispositifs sont bien connus de l'homme de l'art et comprennent les pompes non-aérosols ou "atomiseurs", les récipients aérosols comprenant un propulseur ainsi que les pompes aérosols utilisant l'air comprimé comme propulseur.

Les compositions et les flacons de l'invention peuvent être sous la forme d'eau de toilette, d'eau de Cologne, d'extrait de parfum, d'esprit de parfum ou d'eau de parfum.

Il est aussi décrit, un procédé pour parfumer la peau ou les cheveux d'un individu qui consiste à appliquer, à l'aide d'un moyen de pulvérisation, sur la peau ou les cheveux de l'individu, la composition décrite précédemment. La composition peut également être appliquée sur les vêtements également, on préfère l'appliquer directement sur la peau, de préférence sur une partie du corps qui n'est pas le visage.

Il est décrit l'utilisation d'un copolymère de vinylpyrrolidone et d'acétate de vinyle dans une composition parfumante, pour prolonger dans le temps la perception de la note olfactive de ladite composition par l'odorat d'un utilisateur l'ayant appliqué sur un support, cette augmentation étant évaluée par rapport à la même composition ne contenant pas ledit copolymère. Le support sur lequel est déposé le parfum peut être par exemple la peau d'une personne ou un support minéral ou organique, de préférence poreux. La composition est de préférence une solution parfumante sous la forme d'eau de toilette, d'eau de Cologne, d'extrait de parfum, d'esprit de parfum ou d'eau de parfum, appliquée sur la peau, les cheveux ou les vêtements. Le copolymère prolonge avantageusement la perception des notes de tête et des notes de coeur de la note olfactive.

Il est aussi décrit un diffuseur comprenant un support imprégné de la composition parfumante décrite précédemment. Le support peut être minéral ou organique, et de préférence poreux.

Le support sur lequel le parfum de l'invention peut être déposé est par exemple un tissu ou une céramique. Les céramiques peuvent être fabriquées selon les techniques connues de l'homme du métier. Les tissus peuvent être des lingettes utilisées dans le domaine des produits parfumants ou parfumés, ou des feutres.

Le diffuseur peut être obtenu par imprégnation du support avec la composition parfumante par tout moyen connu de l'homme du métier. L'imprégnation peut être ainsi réalisée i) par capillarité après que la composition ait été soit versée goutte à goutte, soit pulvérisée sur le support, ou ii) par trempage du support dans un récipient contenant la composition parfumante.

L'invention sera illustrée plus en détail par les exemples suivants.

### Exemple 1 et Exemples comparatifs : Evaluation de la fidélité olfactive et de la rémanence d'une composition parfumante selon l'invention et d'une composition parfumante de l'art antérieur

On a choisi comme Référence un parfum du commerce de type « floral » de rémanence moyenne.

On a ensuite préparé une composition selon l'invention obtenue par ajout d'un copolymère VP/VA dans la Référence. Le copolymère de vinylpyrrolidone (VP) et d'acétate de vinyl (VA) qui a été utilisé présente un maximum de solubilité, mesurée à 25° C, de 40% en poids dans l"éthanol à 96% v/v (comprenant 4% v/v d'eau). La neutralité olfactive intrinsèque du copolymère a été vérifiée. Un tel copolymère est par exemple commercialisé sous la référence PVP/VA S-630® par la société ISP.

A titre de comparaison, on a testé différents polymères polyvinylpyrrolidone (PVP), en fonction de leurs caractéristiques physiques (longueur de chaîne et réticulation), de leurs caractéristiques chimiques (hydrophilie/lipophilie) et de leurs propriétés sensorielles (odeur et couleur).

Ces polymères ont été introduits dans le parfum du commerce à une concentration, préalablement définie comme la concentration maximale à laquelle le parfum est pulvérisable avec une pompe manuelle.

**Tableau 1 - Concentration du copolymère dans la Référence**

| **Solutions** | **Polymère introduit dans la Référence** | **CARACTÉRISTIQUES PHYSIQUES** | **Concentration du copolymère dans la Référence** |
|---|---|---|---|
| Solution 1 comparative | PVP | PM : 2,000,000-3,000,000 | 0.5% |
| Solution 2 comparative | PVP | PM : 900,000-1,500,000 | 2% |
| Solution 3 comparative | PVP | PM : 40,000-80,000 | 3% |
| Solution conforme à l'invention | Copolymère VP/VA | PM : 30,000-60,000 Ratio massique VP/VA = 60/40 | 4% |

| | | | |
|---|---|---|---|
| PM : Masse moléculaire en poids du polymère | | | |

La fidélité olfactive des Solutions a été évaluée par rapport à la Référence par un panel sensoriel composé de 8 personnes, immédiatement après application sur la peau (T0) et 8 heures après application (T0+8 heures), en suivant un protocole d'étalement précis. Le score a été donné sur une échelle de 0 à 9, 9 représentant la fidélité optimale. Un protocole similaire a été utilisé pour déterminer la rémanence des Solutions à T0+8 heures, notée sur une échelle de 0 à 9, où 9 représente la note la plus favorable.

Les membres du panel sensoriel étaient des experts dotés i) d'une expérience de caractérisations objectives et descriptives des qualités sensorielles de compositions cosmétiques parfumées ou parfumantes, et ii) d'une facilité à faire abstraction des composantes hédoniques.

Les experts du panel ont été instruits sur la manière d'attribuer des notes et de juger les différences. En outre, les experts ne devaient pas se parfumer ni appliquer une crème parfumée les jours d'évaluation.

Le protocole suivi par chaque expert était le suivant :
✔ On pulvérise une première fois la Solution sans l'appliquer pour bien amorcer la pompe.
✔ On applique une seule pulvérisation de la Solution, à environ 5 cm du bras, sur la peau de la partie supérieure haute/milieu de l'avant-bras de l'expert, et on réalise la même application avec la Référence sur l'autre avant-bras.
✔ On évalue la fidélité olfactive à T0 sur une échelle de 0 à 9 en comparant les notes parfumées de la Référence et de la Solution.
✔ On applique un cercle en plastique autour de chacune des deux zones de peau parfumées pour les protéger des frottements ; on les fixe avec un sparadrap et on attend 8 heures.
✔ A T0+8 heures, on retire les deux cercles de plastique, et après un 10 secondes, on sent les zones parfumées à environ 2 cm de la surface de la peau et on évalue la rémanence ou la fidélité olfactive de la Solution sur une échelle de 0 à 9.

Les résultats ont été reportés dans les tableaux 2 et 3 ci-dessous.

**Tableau 2- Fidélité olfactive à T0**

| **Solutions** | **POURCENTAGE** | **Fidélité T0 par rapport à la Référence** |
|---|---|---|
| Solution 1 comparative | 0.5% PVP | 6.75 |
| Solution 2 comparative | 2% PVP | 7.1 |
| Solution 3 comparative | 3% PVP | 8 |
| Solution conforme à l'invention | 4% Copolymère VP/VA | 8 |

**Tableau 3- Fidélité et Rémanence à T0+8 heures**

| **INGREDIENTS** | **POURCENTAGE de polymère** | **Rémanence T0+8H** | **Fidélité T0+8H** |
|---|---|---|---|
| Référence | 0% | 5 | - |
| Solution 1 comparative | 0.5% PVP | 5.4 | 6.4 |
| Solution 2 comparative | 2% PVP | 6.2 | 6.6 |
| Solution 3 comparative | 3% PVP | 4.8 | 7.5 |
| Solution de l'invention | 4% Copolymère VP/VA | 6.4 | 7.5 |

Cette étude indique que seul le copolymère VP/VA permet d'améliorer significativement à la fois la fidélité olfactive et la rémanence du parfum, sans impact sur ses propriétés physiques et sensorielles.

La fidélité de la note olfactive est respectée, l'aptitude à la pulvérisation est préservée, et un bon compromis peut être obtenu entre la sensorialité et la durée du produit après l'application.

### Exemple 2 : Augmentation de la Rémanence de différents parfums

Une étude similaire à celle de l'exemple 1 a été réalisée en solubilisant un copolymère de VP/VA à 4% en poids conforme à celui utilisé dans l'exemple 1, respectivement dans quatre parfums du commerce P1 à P4 ayant des rémanences différentes (voir le Tableau 4). Les résultats ont été présentés dans la Tableau 5.

**Tableau 4- Rémanence des parfums Références**

| Parfum Référence sans polymère | Rémanence T0+8H du parfum sans polymère |
|---|---|
| P1 | 3 |
| P2 | 4 |
| P3 | 5 |
| P4 | 6 |

**Tableau 5- Fidélité olfactive et Rémanence des solutions de l'invention**

| Parfum contenant le copolymère VP/VA | Fidélité Olfactive T0+8H | Rémanence T0+8H | Augmentation de la Rémanence T0+8H |
|---|---|---|---|
| P1 avec 4% de VP/VA | 7.2 | 5.8 | 93% |
| P2 avec 4% de VP/VA | 7.3 | 5.8 | 45% |
| P3 avec 4% de VP/VA | 7.5 | 6.4 | 28% |
| P4 avec 4% de VP/VA | 6.8 | 6.7 | 12% |

L'augmentation de la rémanence à T0+8H est significative quand on ajoute au parfum un copolymère de VP/VA solubilisé à 4% en poids, quelle que soit la famille olfactive du parfum.

L'effet le plus important a été obtenu avec un parfum très léger dont la rémanence était de l'ordre de 3 à 4.

### Exemple 3 : Préparation de céramiques imprégnées de la composition parfumante de l'invention et comparaison par rapport à une imprégnation avec une composition parfumante de l'art antérieur

Des essais d'imprégnation de céramiques ont été réalisés avec le parfum P1 et le parfum Référence sans polymère, ces deux parfums étant conformes à ceux décrits dans l'exemple 2.

Le diffuseur « Témoin » est constitué d'une céramique qui a été parfumée par trempage, en l'immergeant totalement pendant 30 secondes, à 25°C, dans le parfum Référence, le parfum Référence illustrant l'art antérieur et étant dépourvu de copolymère PVP-VA.

Le diffuseur de l'invention» est constitué d'une céramique ayant les mêmes caractéristiques que la céramique du diffuseur témoin,= utilisée précédemment, et qui a été parfumée par trempage dans le parfum P1 conforme à l'invention contenant le copolymère PVP/VA, dans les mêmes conditions de trempage que celles utilisées pour préparer la céramique témoin.

Une note comprise entre 0 et 4 (4 étant la note maximale de rémanence perçue) est attribuée par un panel olfactif conforme à celui utilisé dans l'Exemple 1. Cette note caractérise la rémanence du parfum P1 ou du parfum Référence sur céramique.

Trois notes ont été attribuées :
- à la céramique qui a été trempée et qui vient d'être sortie du bêcher, sans séchage ultérieur (note à T0)
- à la céramique conforme à T0 qui a été ensuite conservée 6 mois à Température ambiante (note à T1), puis
- à la céramique conforme à T1 qui a été ensuite mise en vieillissement accéléré 3 semaines dans une étuve à 45°C (note à T2).

Les notes moyennes attribuées sont reportées dans les Tableau suivant.

| | T0 | T1 = T0 + 6 mois Température ambiante | T2 = T1 + 3 semaines à 45°C |
|---|---|---|---|
| Diffuseur Témoin | 4 | 2 | 3.5 |
| Diffuseur invention | 4 | 1.5 | 3 |

## Revendications

1. Composition parfumante comprenant
- de 40 à 90% en poids d'éthanol,
- de 0 à 30% en poids d'eau,
- de 3 à 40% en poids d'un concentré de parfum, et
- de 1 à 10% en poids d'un copolymère de vinylpyrrolidone et d'acétate de vinyle, le ratio massique entre le vinylpyrrolidone et l'acétate de vinyle dans le copolymère étant égal à 60 :40,
les pourcentages étant exprimés par rapport au poids de la composition.

2. Composition selon la revendication 1, **caractérisée en ce que** le concentré de parfum représente de 10 à 35% en poids, et le copolymère représente de 2 à 5% en poids du poids de la composition.

3. Composition selon l'une quelconque des revendications 1 à 2, **caractérisée en ce que** le copolymère est solide à 25°C et **en ce que** la masse moléculaire en poids du copolymère est comprise entre 15.000 et 600.000.

4. Composition selon l'une quelconque des revendications précédentes, **caractérisée en ce que** le ratio massique entre l'éthanol et l'eau est compris entre 65/35 et 98/2.

5. Composition selon l'une quelconque des revendications 1 à 4, **caractérisée en ce que** la composition contient de 15 à 25% en poids de concentré de parfum, de 55 à 65% en poids d'éthanol et de 15 à 25% en poids d'eau par rapport au poids de la composition.

6. Composition selon l'une quelconque des revendications 1 à 3, **caractérisée en ce que** la composition contient de 25 à 35% de concentré de parfum, de 70 à 80% en poids d'éthanol et 0% en poids d'eau par rapport au poids de la composition.

7. Composition selon l'une quelconque des revendications 1 à 3, **caractérisée en ce que** la composition contient de 15 à 25% en poids de concentré de parfum, de 75 à 85% en poids d'éthanol et de 0 à 5% en poids d'eau par rapport au poids de la composition.

8. Flacon comprenant un moyen de pulvérisation et un moyen de conditionnement, et contenant une composition parfumante selon l'une des revendications précédentes.

9. Flacon selon la revendication 8, **caractérisée en ce que** la composition est transparente et que le flacon est transparent.

10. Flacon selon la revendication 8 ou 9, **caractérisée en ce que** la composition est une solution sous la forme d'eau de toilette, d'eau de Cologne, d'extrait de parfum, d'esprit de parfum ou d'eau de parfum.

11. Flacon selon l'une quelconque des revendications 8 à 10, **caractérisée en ce que** le moyen de pulvérisation est une pompe manuelle.

## Patentansprüche

1. Parfümzusammensetzung, umfassend:
- 40 bis 90 Gew.-% Ethanol,
- 0 bis 30 Gew.-% Wasser,
- 3 bis 40 Gew.-% eines Parfümkonzentrats und
- 1 bis 10 Gew.-% eines Vinylpyrrolidon / Vinylacetat-Copolymers, wobei das Gewichtsverhältnis des Vinylpyrrolidons zu dem Vinylacetat im Copolymer 60:40 beträgt;
die Prozentsätze werden bezogen auf das Gewicht der Zusammensetzung ausgedrückt.

2. Zusammensetzung nach Anspruch 1, **dadurch gekennzeichnet, dass** das Parfümkonzentrat 10 bis 35 Gew.-% ausmacht und das Copolymer 2 bis 5 Gew.-% des Gewichts der Zusammensetzung ausmacht.

3. Zusammensetzung nach einem der Ansprüche 1 bis 3, **dadurch gekennzeichnet, dass** das Copolymer bei 25°C fest ist und das Gewichtsmittel-Molekulargewicht des Copolymers zwischen 15 000 und 600 000 liegt.

4. Zusammensetzung nach einem der vorhergehenden Ansprüche, **dadurch gekennzeichnet, dass** das Gewichtsverhältnis zwischen Ethanol und Wasser zwischen 65/35 und 98/2 liegt.

5. Zusammensetzung nach einem der Ansprüche 1 bis 4, **dadurch gekennzeichnet, dass** die Zusammensetzung 15 bis 25 Gew.-% Parfümkonzentrat, 55 bis 65 Gew.-% Ethanol und 15 bis 25 Gew.-% Wasser bezogen auf das Gewicht der Zusammensetzung enthält.

6. Zusammensetzung nach einem der Ansprüche 1 bis 3, **dadurch gekennzeichnet, dass** die Zusammensetzung 25 bis 35 Gew.-% Parfümkonzentrat, 70 bis 80 Gew.-% Ethanol und 0 Gew.-% Wasser bezogen auf das Gewicht der Zusammensetzung enthält.

7. Zusammensetzung nach einem der Ansprüche 1 bis 3, **dadurch gekennzeichnet, dass** die Zusammensetzung 15 bis 25 Gew.-% Parfümkonzentrat, 75 bis 85 Gew.-% Ethanol und 0 bis 5 Gew.-% Wasser bezogen auf das Gewicht der Zusammensetzung enthält.

8. Flasche, umfassend ein Sprühmittel und ein Verpackungsmittel, und enthaltend eine Parfümzusammensetzung nach einem der vorhergehenden Ansprüche.

9. Die Flasche nach Anspruch 8, **dadurch gekennzeichnet, dass** die Zusammensetzung transparent ist und dass die Flasche transparent ist.

10. Flasche nach Anspruch 8 oder 9, **dadurch gekennzeichnet, dass** die Zusammensetzung eine Lösung in Form von Eau de Toilette, Eau de Cologne, Parfümextrakt, Esprit de Parfum oder Eau de Parfum ist.

11. Flasche nach einem der vorhergehenden Ansprüche 8 bis 10, **dadurch gekennzeichnet, dass** das Sprühmittel eine manuelle Pumpe ist.

## Claims

1. A perfuming composition comprising:
- from 40% to 90% by weight of ethanol,
- from 0% to 30% by weight of water,
- from 3% to 40% by weight of a perfume concentrate, and
- from 1% to 10% by weight of a vinylpyrrolidone/vinyl acetate copolymer, the weight ratio of the vinylpyrrolidone to the vinyl acetate in the copolymer being 60:40,
the percentages being expressed relative to the weight of the composition.

2. The composition as claimed in claim 1, **characterized in that** the perfume concentrate represents from 10% to 35% by weight, and the copolymer represents from 2% to 5% by weight of the weight of the com position.

3. The composition as claimed in any one of claims 1 to 2, **characterized in that** the copolymer is solid at 25°C and **in that** the weight-average molecular weight of the copolymer is between 15 000 and 600 000.

4. The composition as claimed in any one of the preceding claims, **characterized in that** the weight ratio of ethanol to water is between 65/35 and 98/2.

5. The composition as claimed in any one of claims 1 to 4, **characterized in that** the composition contains from 15% to 25% by weight of perfume concentrate, from 55% to 65% by weight of ethanol and from 15% to 25% by weight of water relative to the weight of the composition.

6. The composition as claimed in any one of claims 1 to 3, **characterized in that** the composition contains from 25% to 35% of perfume concentrate, from 70% to 80% by weight of ethanol and 0% by weight of water relative to the weight of the composition.

7. The composition as claimed in any one of claims 1 to 3, **characterized in that** the composition contains from 15% to 25% by weight of perfume concentrate, from 75% to 85% by weight of ethanol and from 0% to 5% by weight of water relative to the weight of the composition.

8. A bottle comprising a spraying means and a packaging means, and containing a perfuming composition as claimed in any one of the preceding claims.

9. The bottle according to claim 8, **characterized in that** the composition is transparent, and **in that** the bottle is transparent.

10. The bottle as claimed in claim 8 or 9, **characterized in that** the composition is a solution in the form of eau de toilette, eau de cologne, perfume extract, esprit de parfum or eau de parfum.

11. The bottle as claimed in any one of claims 8 to 10, **characterized in that** the spraying means is a manual pump.
